# EUROPEAN PATENT SPECIFICATION

(11) **EP 2 189 530 B1**
(45) Date of publication and mention of the grant of the patent: **27.01.2016**
(21) Application number: 09176347.4
(22) Date of filing: 18.11.2009
(51) Int. Cl.: C12N 15/10

(54) **Method of separating genomic DNA and plasmid DNA from each other and kit therefor**
Verfahren zur Trennung genomischer DNA und Plasmid-DNA voneinander und Kit dafür
Procédé de séparation de l'ADN génomique de l'ADN plasmidique et kit correspondant

(30) Priority: 19.11.2008 KR 20080115326
(43) Date of publication of application: 26.05.2010
(73) Proprietor: Samsung Electronics Co., Ltd., Suwon-si, Gyeonggi-do, 443-742 (KR)
(72) Inventor: Lim, Hee-kyun, Gyeonggi-do (KR); Hwang, Kyu-youn, Gyeonggi-do (KR); Kim, Joon-ho, Gyeonggi-do (KR)
(74) Representative: Grünecker Patent- und Rechtsanwälte PartG mbB

(56) References cited:
- EP-A- 0 814 156
- EP-A1- 1 510 577
- EP-A1- 1 715 039
- EP-A1- 1 724 016
- EP-A2- 1 473 299
- EP-A2- 1 674 570
- EP-A2- 1 892 295
- WO-A-02/38758
- WO-A-2008/150826
- WO-A1-95/06652
- WO-A1-2004/055213
- DE-A1- 4 321 904
- DE-A1- 19 858 447
- US-A1- 2001 018 513
- US-B1- 6 787 307
- LEVISON P R ET AL: "New approaches in the binding of DNA for clinical applications." CLINICAL CHEMISTRY SEP 1998, vol. 44, no. 9, September 1998 (1998-09), pages 2060-2061, XP002562928 ISSN: 0009-9147
- SIUN CHEE TAN AND BEOW CHIN YIAP: "DNA, RNA, and Protein Extraction: The Past and The Present" JOURNAL OF BIOMEDICINE AND BIOTECHNOLOGY, [Online] vol. 2009, 5 November 2009 (2009-11-05), XP002562930 Retrieved from the Internet: URL:http://downloads.hindawi.com/journals/ jbb/2009/574398.pdf> [retrieved on 2010-01-13]

## Description

### BACKGROUND

### 1. Field

One or more embodiments relate to a method of separating genome DNA and plasmid DNA from each other.

### 2. Description of the Related Art

Separation of plasmid DNA from samples, particularly, biological samples, needs to be performed in advance for molecular biological assays such as polymerase chain reactions ("PCRs"), sequence analyses, cloning, and transformation. Recently, plasmid DNA ("pDNA") has been widely applied to vaccines, gene therapies, etc. Accordingly, the separation of plasmid DNA is considered to be important not only in research of molecular biology and biological engineering fields, but also in diagnoses, treatments, and medical applications.

Conventionally, pDNA is separated by lysing cells in an alkali condition, adding acid to the cell lysates to prepare weak acidic cell lysates, adding ethanol thereto, and centrifuging the solution at high speed to precipitate genome DNA ("gDNA"). In this regard, the high-speed centrifugation can be performed at a speed of about 13,000 rpm (about 17,900 g) to about 14,000 rpm (about 20,000 g). Even though a commercially available kit of a QIAgen MiniPrep™ DNA (QIAgen) has been used, high-speed centrifugation is also used.

Microfluidic devices have been widely developed as devices used to analyze biological samples with high capacity and high efficiency. Microfluidic devices need to include motors suitable for precisely controlling components such as valves. For example, a servomotor may be used to precisely control mechanical position or other parameters. However, motors used to precisely control mechanical position do not provide sufficient centrifugal force to obtain high-speed centrifugation.

Thus, there is a need to develop alternative methods of efficiently separating gDNA and pDNA from samples. In particular, there is a need to develop methods of separating gDNA and pDNA from samples without using high-speed centrifugation.

### SUMMARY

Disclosed herewith is a method of efficiently separating gDNA and pDNA from a sample using a buffer containing a high concentration kosmotropic salt and chaotropic salt.

Further disclosed is a method of efficiently separating gDNA and pDNA from a sample using a buffer containing a high concentration kosmotropic salt and chaotropic salt and a solid support material capable of binding to DNA.

Disclosed herewith is a kit for efficiently separating gDNA and pDNA from a sample comprising a binding buffer containing a high concentration kosmotropic salt and chaotropic salt, and a solid support material capable of binding to DNA.

Additional aspects will be set forth in part in the description which follows and, in part, will be apparent from the description, or may be learned by practice of the presented embodiments.

Further disclosed is a method of separating genomic DNA and plasmid DNA from each other according to claim 1, the method including: mixing a sample including genome DNA and plasmid DNA with a solution including a kosmotropic salt and a chaotropic salt to prepare a mixture having a pH ranging from about 3 to about 5; and separating the genome DNA and plasmid DNA from the mixture.

Also disclosed is an analytical material for separating genome DNA and plasmid DNA from each other in a sample including a kosmotropic salt, a chaotropic salt, and a solid support material that binds to DNA at a pH of about 3 to about 5.
To achieve the above and/or other aspects, further disclosed is a method of separating genomic DNA and plasmid DNA from each other, the method comprising mixing a sample comprising genome DNA and plasmid DNA with a solution comprising a kosmotropic salt and a chaotropic salt to prepare a mixture having a pH ranging from about 3 to about 5; contacting the mixture with a solid support material capable of binding to genome DNA to form a genome DNA-solid support material complex; separating the genome DNA-solid support material complex from the mixture; and separating the plasmid DNAfrom the remaining mixture from which the genome DNA-solid support material complex is separated, wherein the separating the plasmid DNA from the remaining mixture is performed by contacting the remaining mixture with a metal oxide, separating the metal oxide from the remaining mixture, and eluting plasmid DNA from the separated metal oxide.

WO 02/38758 A and US 6,787,307 B1 relate to a method using a modified silica matrix to clear a solution of disrupted biological material, such as cell lysates or homogenates of plant or animal tissue. The method can be used to isolate a target nucleic acid including, but not limited to, plasmid DNA, DNA fragments, total RNA from a variety of contaminants, including non-target nucleic acids, animal tissue, blood cells or plant material. The modified matrix may comprise a silica solid phase with a plurality of silane ligands attached thereto. Said matrix is combined with a solution comprising disrupted biological material, a target nucleic acid material, and a chaotropic salt at a concentration sufficient to promote selected adsorption of the disrupted biological material to the matrix for clearing the matrix from the disrupted biological material.

Levison, P. R., et al.: "New approaches in the binding of DNA for clinical applications. "CLINICAL CHEMISTRY Sep. 1998, vol. 44, no. 9, September 1998, pages 2060-2061 discloses a method for isolating plasmid DNA using a silica matrix or a silica disc. According to said method, cells are lysed by alkali treatment. Subsequently, genomic DNA and other contaminants are precipitated by addition of 0.75 mol/L potassium acetate (pH 4.6) containing 4 mol/L guanidine hydrochloride. The mixture is centrifuged at 7500 g for 5 minutes to sediment the precipitated protein, cell debris, and denatured chromosomal DNA. Subsequently, the plasmid DNA is isolated using the silica matrix or the silica disc.

EP 0 814 156 A discloses a method for collecting DNA by lysing microbial cells, adsorbing released DNA on a carrier and collecting the DNA adsorbed on the carrier. For neutralizing the alkaline lysis solution and adsorbing the DNA to the carrier, a solution containing a neutralizer and chaotropic ions is used.

WO 2008/150826 A refers to a method for the isolation of plasmid DNA from plasmid-containing cells. The method comprises inter alia the steps of incubating a suspension of plasmid containing cells with a lysis-denaturation solution to lyse the cells and denature DNA; neutralize the mixture with a renaturation solution to generate a renatured mixture of dissolved plasmid DNA and flocculants containing insoluble genomic DNA and cellular debris, and passing this mixture on a modified spin column to remove the flocculents and then passing the mixture to the column matrix.

DE 43 21 904 A1 discloses a method for chromatographically purifying and separating a mixture of nucleic acids by adsorption of said nucleic acids from a solution having a high ionic strength to a carrier, following by desorption of the nucleic acids from the carrier using a solution having a lower salt concentration. The nucleic acids are adsorbed in the presence of chaotropic salts in a high concentration and desorbed with solutions having a lower ionic strength, allowing for a good fractionation of a mixture of nucleic acids. Subsequently, the solution is centrifuged for removing cell debris and precipitated SDS.

EP 1 892 295 A2 relates to a method and a device for isolating and amplifying a nucleic acid from a microorganism cell using nonplanar solid substrates. According to this isolation method, a cell containing a sample is contacted with the nonplanar solid substrate in a liquid medium having a pH of 3.0 to 6.0 for binding the salts to the nonplanar solid substrate, the cells bound to the substrate are subsequently lysed and the nucleic acids released are isolated. The nonplanar solid substrate preferable has a hydrophobic surface having a water contact angle of 70° to 95°.

WO 95/06652 A1 concerns compositions and methods for isolating nucleic acids with a length greater than about 50 bases, from cells, gels and other media in which nucleic acids occur *in vivo* or *in vitro.* The methods are suitable for separating vector DNA, such as plasmid DNA from genomic DNA. The compositions are mixtures of silica gel and glass particles in aqueous solutions of chaotropic salts, such as guanidine chloride. In the aqueous solution, the nucleic acid binds to the silica material.

EP 1 510 577 A1 discloses a method for isolating nucleic acids from other substances in a medium using silica magnetic particles. The target material is preferably DNA or RNA including total DNA, genomic DNA, plasmid DNA or DNA fragments produced from restriction enzyme digestion. In the method, a complex is formed between the magnetic particles, preferably silicious-oxide coated magnetic particles, and the biological material in a medium containing a chaotropic salt.

EP 1 473 299 A2, WO 2004/055213 A1 and US 2001/018513 A1 all relate to the isolation of nucleic acids using a charge switching material comprising an ionisable group, which changes charge according to the ambient conditions, such as the pH of the solution. Charge switching materials are used for binding nucleic acids by contacting a sample with a charge switching material at a first pH, at which the charge switching material has a positive charge, and releasing the nucleic acid at a second, higher pH at which the charge switching material possesses a neutral, negative or less-positive charge than at the first pH.

EP 1 724 016 A1, EP 1 715 039 A1 and EP 1 674 520 A2 relate to the isolation of nucleic acid using a bi-functional material containing a carboxyl group and an amino group so that the material is positively charged to the first pH to allow binding of the nucleic acid and extraction of the nucleic acid is possible at the second pH that is higher than the first pH.

### BRIEF DESCRIPTION OF THE DRAWINGS

These and/or other aspects will become apparent and more readily appreciated from the following description, taken in conjunction with the accompanying drawings of which:
FIG. 1 is an electrophoretogram illustrating DNA separated from controls 1 and 2 according to Example 1;
FIG. 2 is an electrophoretogram illustrating gDNA and pDNA separated using a binding buffer containing a high concentration kosmotropic salt and chaotropic salt;

FIG. 3 is an electrophoretogram illustrating the influence of types of kosmotropic salts on the separation of gDNA and pDNA.

### DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description.

As used herein, the term "and/or" includes any and all combinations of one or more of the associated listed items.

It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the invention.

Spatially relative terms, such as "under" and the like, may be used herein for ease of description to describe the relationship of one element or feature to another element(s) or feature(s) as illustrated in the figures. It will be understood that the spatially relative terms are intended to encompass different orientations of the device in use or operation, in addition to the orientation depicted in the figures. For example, if the device in the figures is turned over, elements described as "under" relative to other elements or features would then be oriented "above" relative to the other elements or features. Thus, the exemplary term "under" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the invention as used herein.

Hereinafter, the invention will be described in detail with reference to the accompanying drawings.

One or more embodiments include a method of separating genomic DNA (gDNA) and plasmid DNA (pDNA) from each other according to claim 1, the method including: mixing a sample including gDNA and pDNA with a solution including a kosmotropic salt and a chaotropic salt to prepare a mixture having a pH ranging from about 3 to about 5 and selectively separating the genome DNA from the mixture, wherein selectively separating the genome DNA from the mixture comprises: contacting the mixture with a solid support material capable of binding to genome DNA and separating the solid support from the mixture.

Preferred embodiments of the invention are presented in the dependent claims.

According to the method, a sample including gDNA and pDNA is mixed with a solution including a kosmotropic salt and a chaotropic salt to prepare a mixture having a pH ranging from about 3 to about 5.

As used herein, the term "genome DNA" refers to DNA forming all or part of genomes of prokaryotic cells or eukaryotic cells. As used herein, the term "plasmid DNA" refers to an extra-chromosomal, independently replicating, circular DNA. The gDNA may be a genome of bacteria, and the pDNA may be plasmid derived from bacteria.

The sample may be any sample including gDNA and pDNA. For example, the sample may be a lysed biological sample. The lysed biological sample may be prokaryotic cell lysates, eukaryotic cell lysates, or a mixture thereof. For example, the lysed biological sample may be one selected from a group consisting of bacteria cell lysates, mold cell lysates, plant cell lysates, and animal cell lysates.

The lysed biological sample may be prepared using known methods, for example, ultrasonic treatment, thermal treatment, pressure treatment, enzyme treatment, and chemical treatment (e.g., alkali treatment). For example, the sample may be cell lysates obtained by treating lysed bacteria cells at a pH ranging from about 12 to about 13.

The "kosmotropic salt" and "chaotropic salt" used herein are materials that change secondary, tertiary, and/or quaternary structures of protein and/or nucleic acid but do not change a primary structure of the protein and/or nucleic acid. Solutes are defined as kosmotropic if they contribute to the stability and structure of water-water interactions. Kosmotropes cause water molecules to favorably interact, which also stabilizes intermolecular interactions in macromolecules. Thus, the kosmotropic salt stabilizes a subject material. In contrast, a chaotropic agent, is a substance which disrupts the three dimensional structure in macromolecules such as proteins, DNA, or RNA and denatures them. Thus, the chaotropic salt destabilizes a subject material. The kosmotropic salt and the chaotropic salt are related to the Hofmeister series according to the degree of influencing solubility of protein in a solution. According to the Hofmeister series, ions are classified into anionic series and cationic series according to their ability to change water structure. The anionic series are as follows:

SO₄²⁻<HPO₄²⁻<OH⁻<F⁻<HCOO⁻<CH₃COO⁻<Cl⁻<Br⁻<NO₃⁻<I⁻<SCN⁻<ClO₄⁻.

The cationic series are as follows:

NH₄⁺, Rb⁺, K⁺, Na⁺, Cs⁺, Li⁺, Ca²⁺, Mg²⁺, and Ba²⁺.

The kosmotropic salt may consist of an anion of the Hofmeister series that stabilizes water structure and its counter cation. The counter cation may be a cationic series of the Hofmeister series. The kosmotropic salt may be a salt consisting of an anion selected from a group consisting of sulfate (SO₄²⁻), phosphate (HPO₄²⁻), hydroxide (OH-), fluoride (F-), formate (HCOO-), and acetate (CH₃COO-), and its cation, but is not limited thereto. The kosmotropic salt induces crystallization of protein, functions as a salting-out ion for hydrophobic particles, and forms a water structure according to the Hofmeister series. The kosmotropic salt may include at least one anion selected from a group consisting of acetate, phosphate, sulfate, and citrate. For example, the kosmotropic salt may be potassium acetate or sodium acetate. The concentration of the kosmotropic salt may be in a range of about 0.3 to about 3 M.

The chaotropic salt may consist of an anion of the Hofmeister series that destabilizes water structure, and its counter cation. The counter cation may be a cationic series of the Hofmeister series. The chaotropic salt may consist of an anion selected from a group consisting of Cl⁻,Br⁻, NO₃⁻, I⁻, SCN⁻, and ClO₄⁻, and its counter cation. The chaotropic salt may be guanidine hydrochloride, guanidine isocyanate, sodium iodide, or guanidine thiocyanate. The concentration of the chaotropic salt may be in a range of about 3 to about 6 M.

However, the kosmotropic salt and the chaotropic salt are not limited to the anions and their counter cations of the Hofmeister series. In this regard, any material stabilizing or destabilizing the structure of protein and/or a nucleic acid may be used.

According to the method of the present embodiment, a molar concentration ratio of the kosmotropic salt to the chaotropic salt may be in a range of about 1: 14 to about 1: 2. The pH of the solution including the kosmotropic salt and the chaotropic salt may be in a range of about 3 to about 5.

For example, in the solution including the kosmotropic salt and the chaotropic salt, the concentration of the kosmotropic salt may be in a range of about 0.3 to 3 M, the concentration of the chaotropic salt may be in a range of about 3 to about 6 M, and a molar concentration ratio of the kosmotropic salt to the chaotropic salt may be in a range of about 1:14 to about 1:2

The method includes separating gDNA and pDNA from the mixture. The "separating gDNA and pDNA" used herein indicates selectively separating either the gDNA or the pDNA from the mixture. In this regard, the separating gDNA and pDNA includes not only purifying each of the separated gDNA and pDNA but also relatively increasing the concentration of each of the gDNA and pDNA. That is, the separating gDNA and pDNA may include purifying or concentrating either the gDNA or the pDNA. According to the concentration, the molar concentration of the gDNA and pDNA may be increased by 50%, 70%, or 90% from the initial concentration of either the gDNA or pDNA.

The separating operation includes contacting the mixture with a solid support and separating the solid support from the mixture.

The solid support is a bi-functional material.

The bi-functional material is positively charged and capable of binding to nucleic acids at a first pH and negatively charged and capable of releasing the bound nucleic acids at a second pH. The bi-functional material may be immobilized to the solid support. The first pH is in a range of about 2 to about 5, for example, about 3 to about 4.5 and about 3.5 to about 4.5. The second pH is in a range of about 7 to about 12, for example, about 7 to about 11, about 8 to about 11, or about 8 to about 10.

The bi-functional material is a polymer including at least one monomer selected from a group consisting of monomers represented by Formula M0, M1, M2, and M3 below, wherein the polymer includes at least one monomer having A moiety and at least one monomer having B moiety.

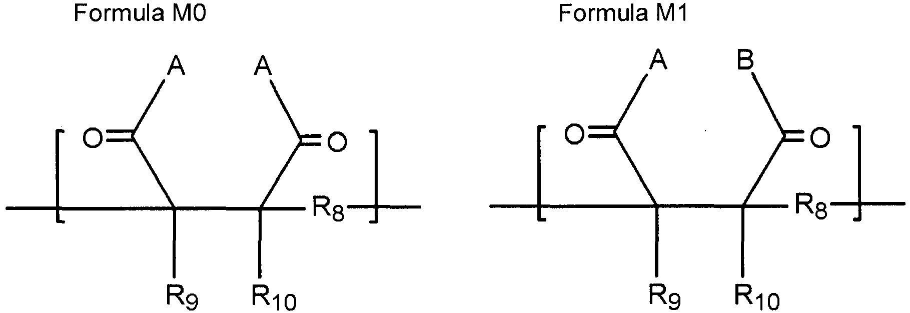

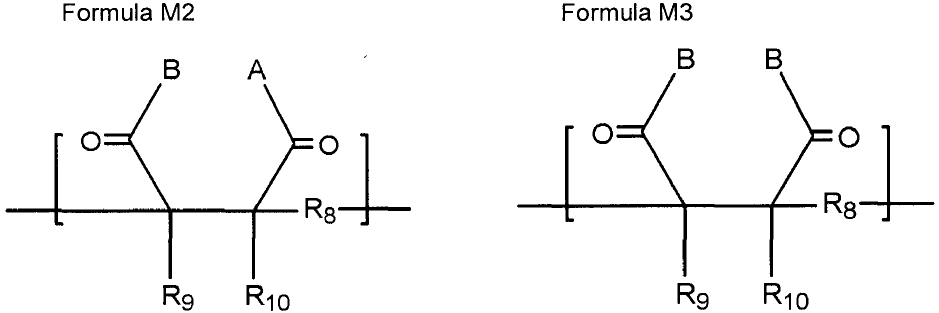

Here, A is a group is -OH and B is

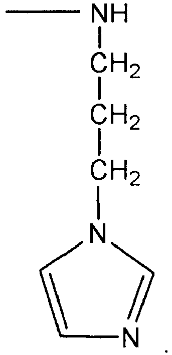

R₈ is ethyl and R₉ and R₁₀ are each hydrogen. A and B may have a polymerization degree of about 2 to about 30,000

In one embodiment, the compound including a monomer having A moiety and a monomer having B moiety may have a polymerization degree of about 2 to about 30.

In one embodiment, the compound including a monomer having A moiety and a monomer having B moiety may be prepared by hydrolyzing a polyanhydride (for example, poly(ethylene-alt-maleic anhydride) (molecular weight: 100,000-500,000) to expose a carboxyl group, reacting the carboxyl group with a material such as, N-hydroxysuccineimide or 1,[3-(dimethylamino)propyl]-3-ethylcarbodiimide to activate the carboxyl group by an ester bond, and then coupling-reacting the activated carboxyl group with A (for example, H₂O) or B (for example, 1-(3-aminopropyl)imidazole). Alternatively, the compound including a monomer having A moiety and a monomer having B moiety may be prepared by directly reacting a polyanhydride (for example, poly(ethylene-alt-maleic anhydride) (molecular weight: 100,000-500,000) with a reactant including A (for example, H₂O) or a reactant including B (for example, 1-(3-aminopropyl)imidazole). In this regard, a net charge of the compound including a monomer having A moiety and a monomer having B moiety may be adjusted by having A or B contained in the compound at an appropriate ratio in the reaction.

The solid support material may be in the form of a bead, a sphere, a plate, a pillar, a sieve, a filter, gel, a membrane, a fiber, a tube, a well, or a channel in a microfluidic device. The solid support material may be magnetic. The solid support material may be formed of glass, silica, latex, or a polymerizable material.

The separation of the solid support material from the mixture may be performed using known methods, such as centrifugation or magnetic force when the solid support is magnetic. The centrifugation may be performed at a low centrifugal force. For example, the low centrifugal force centrifugation may be performed at a force of about 1,500 to about 6,000 g.

In one embodiment, the method may further include separating gDNA from the separated gDNA-solid support material complex. The separation of gDNAfrom the solid support material may be performed by eluting the gDNA from the gDNA-solid support material complex. The elution may be performed at a temperature ranging from about 25 to about 70°. For example, the elution may be performed at 65° at a pH of 8.9 in the presence of 10 mM Tris HCl.

The method may further include separating pDNA from the remaining mixture from which the solid support material is removed. The separation of the pDNA may be performed by contacting the remaining mixture with a solid support material capable of binding to pDNA to form a pDNA-solid support complex, and eluting pDNA from the complex. The solid support may be a metal oxide, such as alumina, a titanium oxide, or a material having SiO₂ structure (e.g., silica). The separation of the pDNA may be performed by contacting the solution with metal oxide, e.g. silica, separating the metal oxide, e.g. silica, from the solution, and eluting pDNA from the separated metal oxide, e.g. silica. The contact may be performed in a solution including kosmotropic salt in a concentration of about 0.3 to about 3M and chaotropic salt in a concentration of about 3 to about 6M at a pH ranging from about 3 to about 5. The metal oxide, e.g. silica, may be separated from the solution using gravity or centrifugal force. The centrifugation may be performed at about 1,500 to about 6,000 g. The elution may be performed at a temperature ranging from about 25 to about 70°. For example, the elution may be performed at 65° at pH 8.9 in the presence of 10 mM Tris HCl.

Further disclosed is an analytical material for separating gDNA and pDNA from each other in a sample including a kosmotropic salt, a chaotropic salt, and a material that binds to DNA at a pH of about 3 to about 5. The kosmotropic salt and the chaotropic salt are as described above.

The analytical material may be a kit. The concentration of the kosmotropic salt may be equal to or greater than 0.3 M, for example, in a range of about 0.3 to about 3 M.

When the chaotropic salt is mixed with the sample, the concentration of the chaotropic salt may be equal to or greater than 3M, for example, in a range of about 3 to about 6 M.

In the analytical material, the concentration of the kosmotropic salt may be in a range of about 0.3 to about 3 M, and the concentration of the chaotropic salt may be in a range of about 3 to about 6 M.

In one embodiment, the analytical material comprises a material that binds to DNA at a pH of about 3 to about 5, which is a bi-functional materialas described above.

The analytical material may be accompanied by a manual explaining a process of separating gDNA and pDNA from each other in a sample. In the manual, the method of separating gDNA and pDNA as described above may be described.

The analytical material may further include a metal oxide, such as alumina, titanium oxide, or a material having a SiO₂ structure (e.g., silica). The metal oxide, e.g., silica, is described above. The metal oxide, e.g., silica, may be used to selectively bind gDNA to a material binding to DNA at a pH ranging from about 3 to about 5, and selectively separate pDNA remaining in a supernatant. The use of the metal oxide, e.g., silica is described above with reference to the separation of gDNA and pDNA.

Hereinafter, embodiments will be described in detail with reference to the following example. However, the example is not intended to limit the purpose and scope of the invention.

### Example 1: Separation of gDNA and pDNA using high concentration kosmotropic salt, chaotropic salt, bi-functional material, and silica

For this example, pDNA and gDNA were separated from bacteria lysates.

First, *E. coli* transformed with pET-21 b plasmid (4.5 kb, Novagen) was cultured in a LB medium containing 50 µg/µl of ampicillin at 37° for 16 hours while shaking at 200 rpm. The culture solution (OD₆₀₀ =1.0) was centrifuged at 8,000 rpm for 10 minutes to obtain *E. coli* pellets. Next, 250 µl of a resuspension buffer (50 mM Tris HCl, 10 mM EDTA, 100 µg/ml RNaseA; pH 8.0) was added to a tube containing the pellets to resuspend the *E*. *coli* pellets.

250 µl of a lysis buffer (200 mM NaOH 1% SDS (w/v); pH 12 to 12.5) was added to the *E. coli* suspension to lyse cells. Then, 100 µl of a binding buffer (0.9 M potassium acetate and 4.2 M guanidine hydrochloride; pH 3.4 to 4.5) was added to the obtained cell lysates.

Then, 0.015 g of bi-functional material beads, discussed in detail below, was added to the cell lysate mixture and the mixture was left to sit at room temperature for 3 to 5 minutes to bind DNA with the bi-functional material beads. Then, the mixture was centrifuged at 3,000 rpm for 3 minutes to separate the bi-functional material beads from a supernatant. Next, 0.015 g of silica beads (SUNSIL-130, average diameter: 15.0 µm, Sunjin Chemical Co., Ltd.) was added to the supernatant from which the bi-functional material beads were removed, and the solution was left to sit at room temperature for 3 to 5 minutes to bind DNA to the silica beads.

Finally, 100 µl of an elution buffer (10 mM Tris HCl, pH 8.5) was added to the silica beads, and the solution was left to sit at 65° for 3 to 5 minutes to elute DNA. In addition, 100 µl of an elution buffer (10 mM Tris HCl, pH 8.9) was added to the bi-functional material beads, and the solution was left to sit at 65° for 3 to 5 minutes to elute DNA. Nucleic acids in the solution were identified using electrophoresis in 0.8% agarose.

As control 1, DNA was separated using a commercially available bacteria gDNA separation kit, QIAamp DNA Mini Kit™ (Qiagen™, Catalog No. 51304) according to a manual (QIAamp DNA Mini™ and Blood Mini™ Handbook, 2nd edition, November 2007).

As control 2, DNA was separated using a binding buffer having different salt concentrations, and bi-functional material beads. For example, 250 µl of a lysis buffer (200 mM NaOH 1% SDS (w/v): pH 12 to 12.5) was added to an *E. coli* suspension to lyse the cells. Then, 1 ml of a binding buffer (100 mM sodium acetate buffer, pH 4) was added to the obtained cell lysates. Then, 0.015 g of bi-functional material beads were added to the cell lysate mixture and the mixture was left to sit at room temperature for 3 to 5 minutes and centrifuged at 3,000 rpm for 3 minutes to separate the bi-functional material beads from the supernatant. The separated bi-functional material beads were suspended in 10 mM Tris-HCl at pH 7, and the suspension was centrifuged at 3,000 rpm for 3 minutes to wash the bi-functional material beads. The washed bi-functional material beads were suspended in 100 mM Tris-HCl buffer at pH 9, and left to sit at 65° for 3 to 5 minutes to elute DNA. Then, the resultant was centrifuged at 3,000 rpm for 3 minutes to separate the bi-functional material beads from the solution to obtain a supernatant containing DNA. Nucleic acids in the solution were identified using electrophoresis in 0.8% agarose.

For this example, the bi-functional material beads were prepared using the following process. First, poly(ethylene-alt-maleic anhydride) (average molecular weight: 100,000-500,000; n=900-4,000) were immobilized onto magnetic beads (Invitrogen Dyanl AS, Catalog No. 161-02, Dynabeads^{®} M-270 Amine, 2x10⁹ beads/ml, diameter: 2.8 µm) coated with an amino group, and the beads were subjected to reaction with 1-(3-aminopropyl)imidazole to prepare a material having a carboxyl group and an imidazole group and positively charged at a first pH and negatively charged at a second pH. Here, the first pH was in a range of about 2 to about 5, and the second pH was in a range of about 7 to about 12.

The magnetic beads coated with the amino group were immersed in 200 mM of poly(ethylene-alt-maleic anhydride (average molecular weight 100,000-500,000, n= 900-4,000) based on a repeating unit of ethylene-maleic anhydride in N-methyl-2-pyrrolidone ("NMP") and cultured at room temperature for 1 hour. As a result, poly(ethylene-alt-maleic anhydride) was subjected to reaction with the amino group of the magnetic beads to be fixed on a substrate. After the reaction, the magnetic beads were washed with ethanol and dried.

Then, the magnetic beads to which the polyanhydride (poly(ethylene-altmaleic anhydride) was immobilized were incubated in 400 mM 1-(3-aminopropyl)imidazole solution and 600 mM water solution in N-methyl-2-pyrrolidone ("NMP") at room temperature for 1 hour. Then, the beads were washed with ethanol. As a result, a compound having a carboxyl group and an imidazole group and positively charged at a first pH and negatively charged at a second pH was prepared. The obtained compound was washed with ethanol and dried.

The obtained compound was a bi-functional compound bound to at least one monomer selected from a group consisting of monomers represented by Formulas M0, M1, M2, and M3 below including at least monomer having A moiety and at least monomer having B moiety.

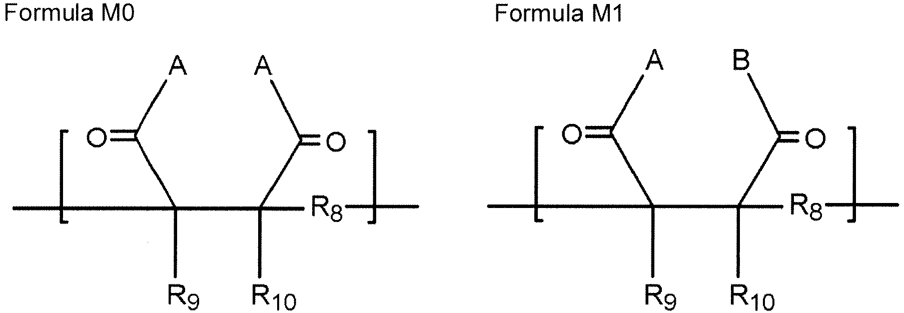

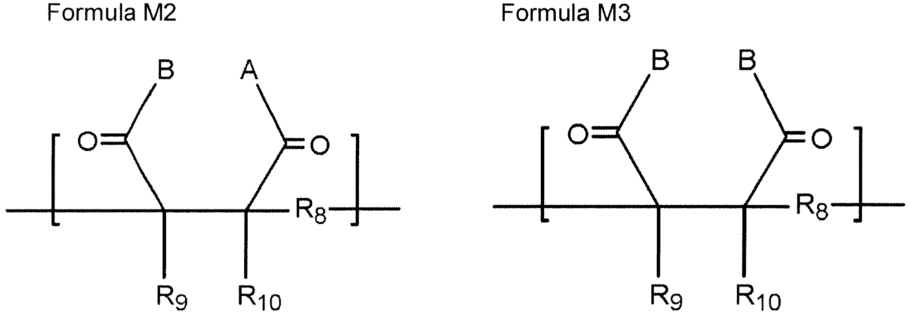

Here, A is -OH, B is 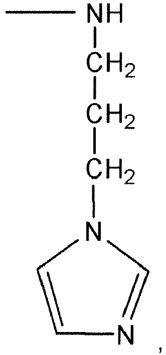 R₈ is ethyl, and R₉ and R₁₀ are hydrogen.

XPS analysis and TOF-SIMS analysis of the surface of the beads to which the prepared compound was immobilized were performed as described in Example 1. X-ray photoelectron spectroscopy (XPS) is a quantitative spectroscopic technique that measures the elemental composition, empirical formula, chemical state and electronic state of the elements that exist within a material. XPS spectra are obtained by irradiating a material with a beam of X-rays while simultaneously measuring the kinetic energy (KE) and number of electrons that escape from the top 1 to 10 nm of the material being analyzed. A typical XPS spectrum is a plot of the number of electrons detected (sometimes per unit time) (Y-axis, ordinate) versus the binding energy of the electrons detected (X-axis, abscissa). Each element produces a characteristic set of XPS peaks at characteristic binding energy values that directly identify each element that exist in or on the surface of the material being analyzed, Time-of-Flight Secondary Ion Mass Spectrometry (TOF-SIMS) uses a pulsed primary ion beam to desorb and ionize species from a sample surface. The resulting secondary ions are accelerated into a mass spectrometer, where they are mass analyzed by measuring their time-of-flight from the sample surface to the detector. TOF-SIMS provides spectroscopy for characterization of chemical composition, imaging for determining the distribution of chemical species, and depth profiling for thin film characterization. According to the XPS analysis, the compound contains a carbonyl group including -OH. In addition, according to the TOF-SIMS analysis, the existence of an imidazolyl group was identified.

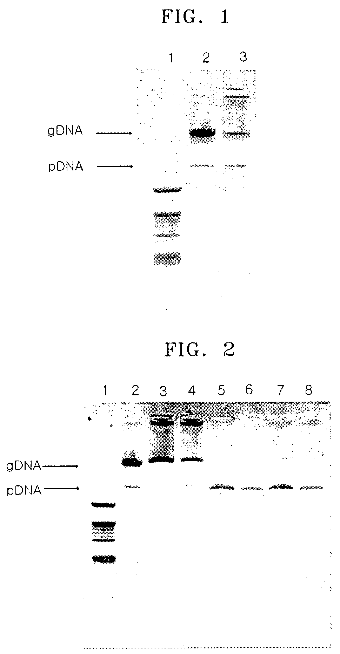

FIG. 1 is an electrophoretogram illustrating DNA separated from controls 1 and 2 according to Example 1. In FIG. 1, lane 1 illustrates results of a DNA ladder (25/100bp mixed DNA ladder, Bioneer, Korea, Catalog No. D-1020), lane 2 illustrates results of control 1, and lane 3 illustrates results of control 2. Referring to FIG. 1, in controls 1 and 2, gDNA and pDNA were identified in the finally eluted solution using an electrophoretogram, and the amount of the gDNA and pDNA was identified with the naked eye. Thus, the efficiency of the gDNA and pDNA separations was not sufficiently high.

FIG. 2 is an electrophoretogram illustrating gDNA and pDNA separated according to Example 1, using a binding buffer containing a high concentration kosmotropic salt and chaotropic salt. In FIG. 2, lane 1 illustrates results of a DNA ladder (25/100bp mixed DNA ladder, Bioneer, Korea, Catalog No. D-1020), lane 2 illustrates results of control 1, lanes 3 and 4 illustrate DNA eluted from the bi-functional material beads, and only pDNA existed in the solution eluted from the silica beads. Thus, gDNA and pDNA were efficiently separated using a binding buffer containing a high concentration kosmotropic salt and chaotropic salt, bi-functional material beads, and silica beads.

Based on the results, since the binding force between gDNA and the bi-functional material was greater than that between pDNA and the bi-functional material in the binding buffer containing a high concentration kosmotropic salt and chaotropic salt, gDNA was separated from pDNA in the form of a gDNA-bi-functional material complex, and then each of gDNA and pDNA was respectively separated from the bi-functional material beads and the solution from which the bi-functional material beads was removed. However, the process of the separation is not limited thereto. ChargeSwitch gDNA Blood Kits; Cat. No. CS11000) were added to the cell lysate mixture, and the mixture was left to sit at room temperature for 3 to 5 minutes to bind DNAto the CS beads. Then, the CS magnetic beads were separated from the supernatant using magnetic force. 0.015 g of silica beads (SUNSIL-130, average diameter: 15.0 µm, Sunjin Chemical Co., Ltd.) were added to the supernatant from which the magnetic CS beads were removed, and the solution was left to sit at room temperature for 3 to 5 minutes to bind DNA to the silica beads.

### Example 2: Influence of kosmotropic salt on the separation of gDNA and pDNA

In order to identify the influence of types of kosmotropic salts on the separation of gDNA and pDNA, the separation was performed in the same manner as in Example 1, except that 0.9 M sodium acetate and 4.2 M guanidine hydrochloride were used as the binding buffer instead of 0.9 M potassium acetate and 4.2 M guanidine hydrochloride.

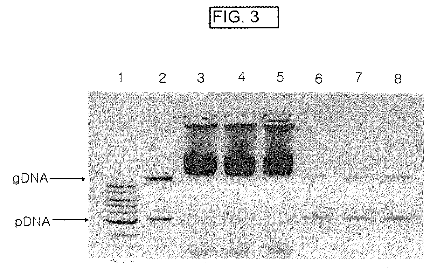

FIG. 3 is an electrophoretogram illustrating the influence of types of kosmotropic salts on the separation of gDNA and pDNA. In FIG. 3, lane 1 illustrates results of a DNA ladder (1 kb DNA ladder, NEB, Catalog No. N-3232S), lane 2 illustrates results of gDNA and pDNA controls (gDNA and pDNA separated from control 1 according to Example 3), lanes 3 and 4 illustrate DNA eluted from the bi-functional material beads using 100 µl of an elution buffer when 0.9 M potassium acetate and 4.2 M guanidine hydrochloride were used as a binding buffer, lane 5 illustrates DNAeluted from the bi-functional material beads using 100 µl of an elution buffer when 0.9 M sodium acetate and 4.2 M guanidine hydrochloride were used as the binding buffer, lanes 6 and 7 illustrate DNA eluted from the silica beads using 100 µl of an elution buffer when 0.9 M potassium acetate and 4.2 M guanidine hydrochloride were used as the binding buffer, lane 8 illustrates DNA eluted from the silica beads using 100 µl of an elution buffer when 0.9 M sodium acetate and 4.2 M guanidine hydrochloride were used as the binding buffer.

As shown in FIG. 3, the results using potassium acetate are similar to those using sodium acetate.

As described above, according to the one or more of the above embodiments, gDNA and pDNA may be efficiently separated from each other using a buffer containing a kosmotropic salt and chaotropic salt, and without using high-speed centrifugation.

## Claims

1. A method of separating genomic DNA and plasmid DNA from each other, the method comprising:
mixing a sample comprising genome DNA and plasmid DNA with a solution comprising a kosmotropic salt and a chaotropic salt to prepare a mixture having a pH ranging from 3 to 5; and selectively separating the genome DNA from the mixture, wherein selectively separating the genome DNA from the mixture comprises:
contacting the mixture with a solid support capable of binding to genome DNA and separating the solid support from the mixture, wherein the solid support material comprises a bi-functional material,
wherein the bi-functional material is positively charged and bound to nucleic acids at a first pH ranging from 2 to 5 and negatively charged and releases the bound nucleic acids at a second pH ranging from 7 to 12, **characterized in that** the bi-functional material is a polymer including at least one monomer selected from a group consisting of monomers represented by Formula M0, M1, M2, and M3 below, wherein the polymer includes at least one monomer having A moiety and at least one monomer having B moiety: 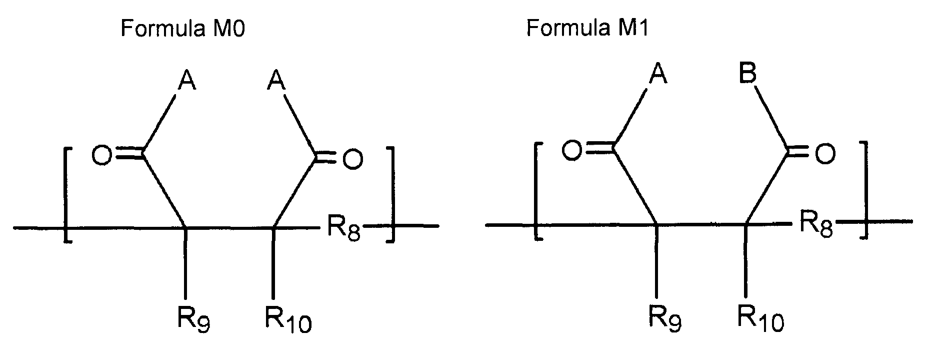 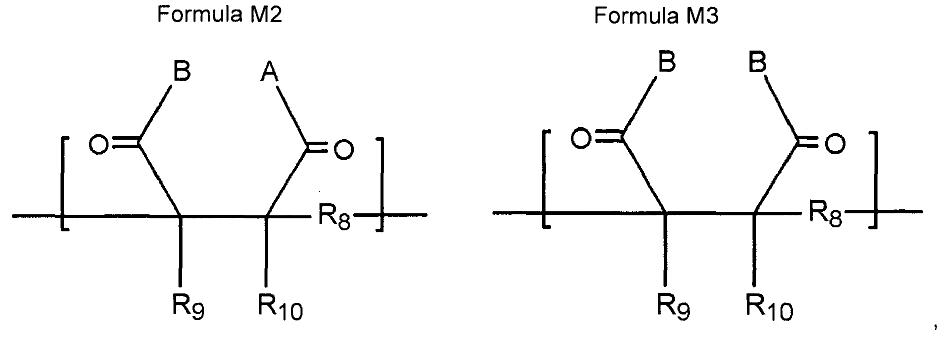 wherein A is -OH, B is 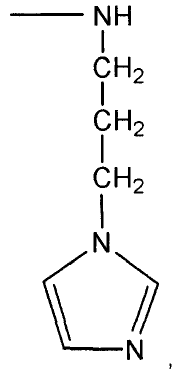 and
wherein R₈ is ethyl, and R₉ and R₁₀ are hydrogen.

2. The method of claim 1, wherein the concentration of the kosmotropic salt is in a range of 0.3 to 3 M, and the concentration of the chaotropic salt is in a range of 3 to 6 M.

3. The method of claim 1 or 2, wherein the kosmotropic salt comprises one anion selected from a group consisting of acetate, phosphate, sulfate, and citrate, and a counter cation of the anion.

4. The method of any one of claims 1 to 3, wherein the chaotropic salt comprises one anion selected from a group consisting of Br⁻, NO₃⁻, I⁻, SCN⁻, and ClO₄⁻, and a counter cation of the anion.

5. The method of any one of claims 1 to 4, wherein a molar concentration ratio of the kosmotropic salt to the chaotropic salt is in a range of 1:14 to 1:2.

6. The method of claim any one of claims 1 to 5, further comprising separating the plasmid DNA from the remaining mixture from which the solid support is separated or from the supernatant by
contacting the supernatant with a metal oxide,
separating the metal oxide from the supernatant, and
eluting plasmid DNA from the separated metal oxide.

7. The method of claim 6, wherein separating the metal oxide from the remaining mixture comprises centrifuging the mixture; and removing the supernatant.

8. The method of any one of claims 1 to 7, wherein the solid-support material further comprises a metal oxide, which metal oxide is preferably a silica material.

9. Use of an analytical material for separating genome DNA and plasmid DNA from each other in a sample comprising
a kosmotropic salt,
a chaotropic salt, and
a material that binds to DNA at a pH of 3 to 5, wherein the material is a bi-functional material, wherein the bi-functional material is positively charged and bound to nucleic acids at a first pH ranging from 2 to 5 and negatively charged and releases the bound nucleic acids at a second pH ranging from 7 to 12, **characterized in that** the bi-functional material is a polymer including at least one monomer selected from a group consisting of monomers represented by Formula M0, M1, M2, and M3 below, wherein the polymer includes at least one monomer having A moiety and at least one monomer having B moiety: 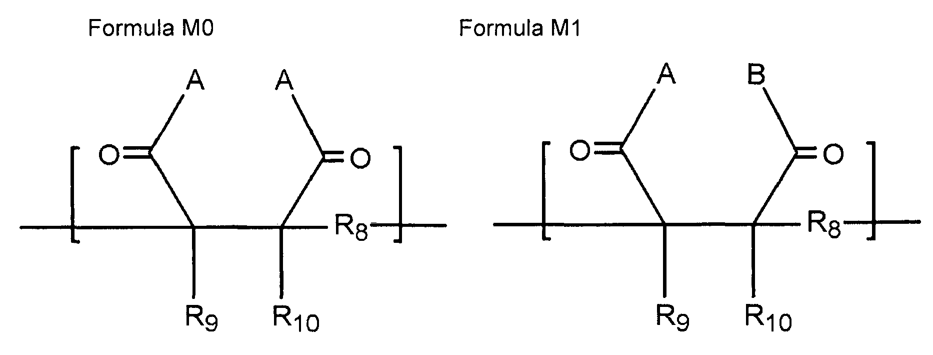 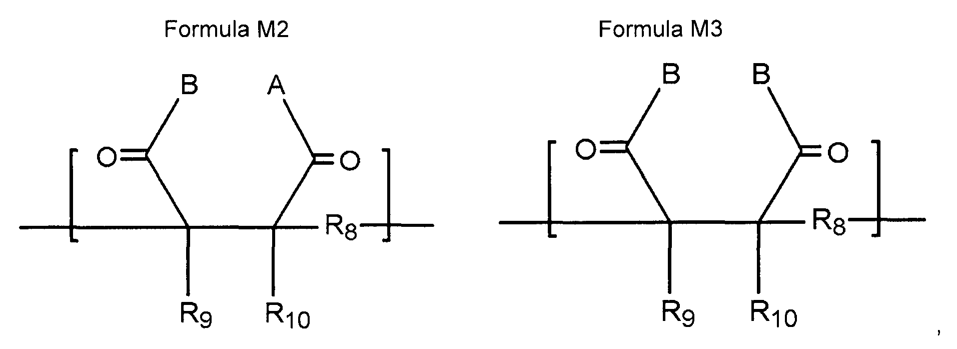 wherein A is -OH, B is 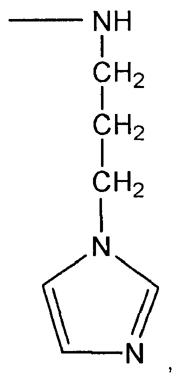 and
wherein R₈ is ethyl, and R₉ and R₁₀ are hydrogen.

10. The use of claim 9, wherein the concentration of the kosmotropic salt is in a range of 0.3 to 3 M, and the concentration of the chaotropic salt is in a range of 3 to 6 M.

11. The use of claim 9 or 10, wherein the analytical material further comprises a metal oxide, which metal oxide is preferably a silica material.

## Patentansprüche

1. Verfahren zum Trennen von genomischer DNA und Plasmid-DNA voneinander, wobei das Verfahren umfasst:
das Mischen einer Probe, die Genom-DNA und Plasmid-DNA umfasst, mit einer Lösung, die ein kosmotropes Salz und ein chaotropes Salz umfasst, um eine Mischung mit einem pH im Bereich von 3 bis 5 herzustellen; und das selektive Trennen der Genom-DNA von der Mischung, wobei das selektive Trennen der Genom-DNA von der Mischung umfasst:
das Inkontaktbringen der Mischung mit einem festen Träger, der an Genom-DNA binden kann, und das Trennen des festen Trägers von der Mischung, wobei das feste Trägermaterial ein bifunktionelles Material umfasst,
wobei das bifunktionelle Material bei einem ersten pH im Bereich von 2 bis 5 positiv geladen und an Nukleinsäuren gebunden ist und bei einem zweiten pH im Bereich von 7 bis 12 negativ geladen ist und die gebundenen Nukleinsäuren freisetzt, **dadurch gekennzeichnet, dass** das bifunktionelle Material ein Polymer ist, das wenigstens ein Monomer, ausgewählt aus einer Gruppe bestehend aus Monomeren, die durch Formel M0, M1, M2 und M3 nachstehend wiedergegeben sind, einschließt, wobei das Polymer wenigstens ein Monomer mit einer A-Einheit und wenigstens ein Monomer mit einer B-Einheit einschließt: 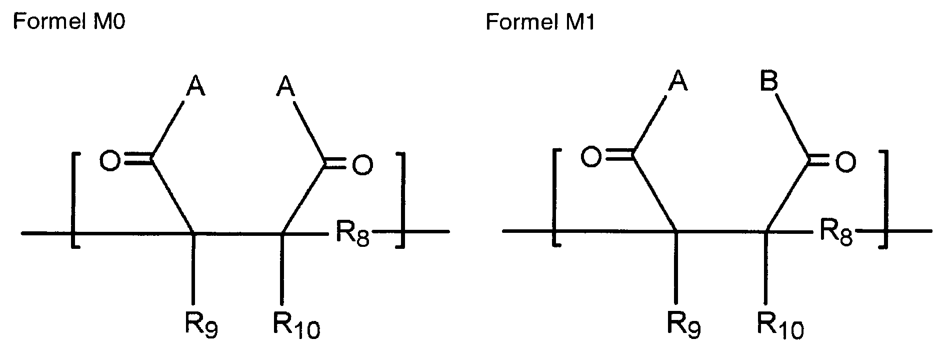 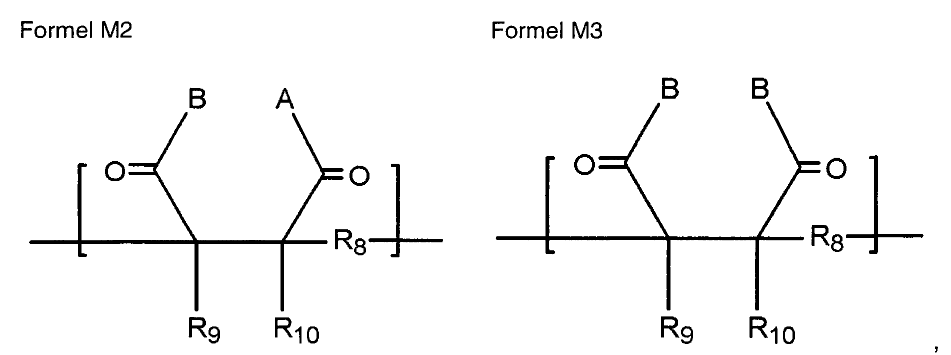 wobei A -OH ist, B 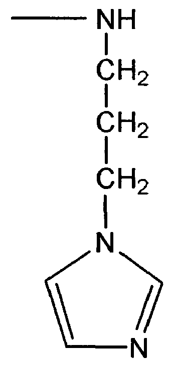 ist, und
wobei R₈ Ethyl ist und R₉ und R₁₀ Wasserstoff sind.

2. Verfahren nach Anspruch 1, wobei die Konzentration des kosmotropen Salzes in einem Bereich von 0,3 bis 3 M liegt und die Konzentration des chaotropen Salzes in einem Bereich von 3 bis 6 M liegt.

3. Verfahren nach Anspruch 1 oder 2, wobei das kosmotrope Salz ein Anion, ausgewählt aus einer Gruppe bestehend aus Acetat, Phosphat, Sulfat und Citrat, und ein Gegenkation des Anions umfasst.

4. Verfahren nach einem der Ansprüche 1 bis 3, wobei das chaotrope Salz ein Anion, ausgewählt aus einer Gruppe bestehend aus Br⁻, NO₃⁻, I⁻, SCN⁻ und ClO₄⁻, und ein Gegenkation des Anions umfasst.

5. Verfahren nach einem der Ansprüche 1 bis 4, wobei ein molares Konzentrationsverhältnis des kosmotropen Salzes zu dem chaotropen Salz in einem Bereich von 1:14 bis 1:2 liegt.

6. Verfahren nach einem der Ansprüche 1 bis 5, außerdem umfassend das Trennen der Plasmid-DNA von der verbleibenden Mischung, von welcher der feste Träger abgetrennt ist, oder von dem Überstand durch
das Inkontaktbringen des Überstands mit einem Metalloxid,
das Trennen des Metalloxids von dem Überstand, und
das Eluieren von Plasmid-DNA von dem abgetrennten Metalloxid.

7. Verfahren nach Anspruch 6, wobei das Trennen des Metalloxids von der verbleibenden Mischung das Zentrifugieren der Mischung; und das Entfernen des Überstands umfasst.

8. Verfahren nach einem der Ansprüche 1 bis 7, wobei das feste Trägermaterial außerdem ein Metalloxid umfasst, wobei dieses Metalloxid vorzugsweise ein Siliciumdioxidmaterial ist.

9. Verwendung eines analytischen Materials zum Trennen von Genom-DNA und Plasmid-DNA voneinander in einer Probe umfassend
ein kosmotropes Salz,
ein chaotropes Salz, und
ein Material, welches bei einem pH von 3 bis 5 an DNA bindet, wobei das Material ein bifunktionelles Material ist, wobei das bifunktionelle Material bei einem ersten pH im Bereich von 2 bis 5 positiv geladen und an Nukleinsäuren gebunden ist und bei einem zweiten pH im Bereich von 7 bis 12 negativ geladen ist und die gebundenen Nukleinsäuren freisetzt, **dadurch gekennzeichnet, dass** das bifunktionelle Material ein Polymer ist, das wenigstens ein Monomer, ausgewählt aus einer Gruppe bestehend aus Monomeren, die durch Formel M0, M1, M2 und M3 nachstehend wiedergegeben sind, einschließt, wobei das Polymer wenigstens ein Monomer mit einer A-Einheit und wenigstens ein Monomer mit einer B-Einheit einschließt: 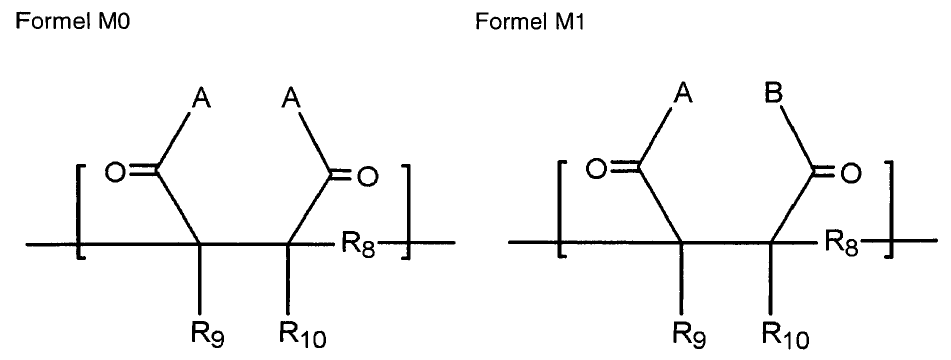 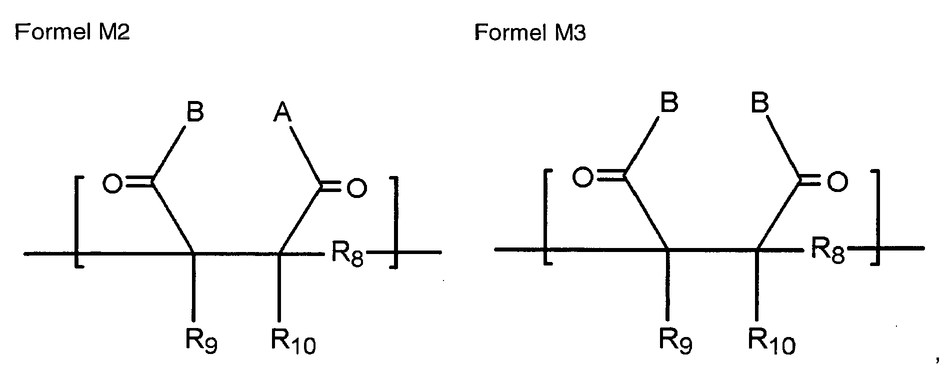 wobei A -OH ist, B 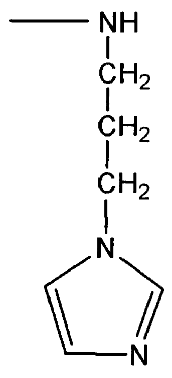 ist, und wobei R₈ Ethyl ist und R₉ und R₁₀ Wasserstoff sind.

10. Verwendung nach Anspruch 9, wobei die Konzentration des kosmotropen Salzes in einem Bereich von 0,3 bis 3M liegt und die Konzentration des chaotropen Salzes in einem Bereich von 3 bis 6 M liegt.

11. Verwendung nach Anspruch 9 oder 10, wobei das analytische Material außerdem ein Metalloxid umfasst, wobei das Metalloxid vorzugsweise ein Siliciumdioxidmaterial ist.

## Revendications

1. Une méthode de séparation de l'ADN génomique de l'ADN plasmidique, la méthode comprenant :
le mélange d'un échantillon comprenant de l'ADN génomique et de l'ADN plasmidique avec une solution comprenant un sel cosmotropique et un sel chaotropique pour préparer un mélange de pH allant de 3 à 5 ; et la séparation sélective de l'ADN génomique du mélange, où la séparation sélective de l'ADN génomique du mélange comprend :
la mise en contact du mélange avec un support solide susceptible de se lier à l'ADN génomique et la séparation du support solide du mélange, où le matériau du support solide comprend un matériau bifonctionnel,
où le matériau bifonctionnel est chargé positivement et lié aux acides nucléiques, à un premier pH allant de 2 à 5, et chargé négativement et relâche les acides nucléiques liés à un deuxième pH allant de 7 à 12, **caractérisé en ce que** le matériau bifonctionnel est un polymère incluant au moins un monomètre sélectionné dans un groupe constitué des monomères représentés par la formule M0, M1, M2 et M3 ci-dessous, où le polymère inclut au moins un monomère comprenant un fragment A et au moins un monomère comprenant un fragment B : 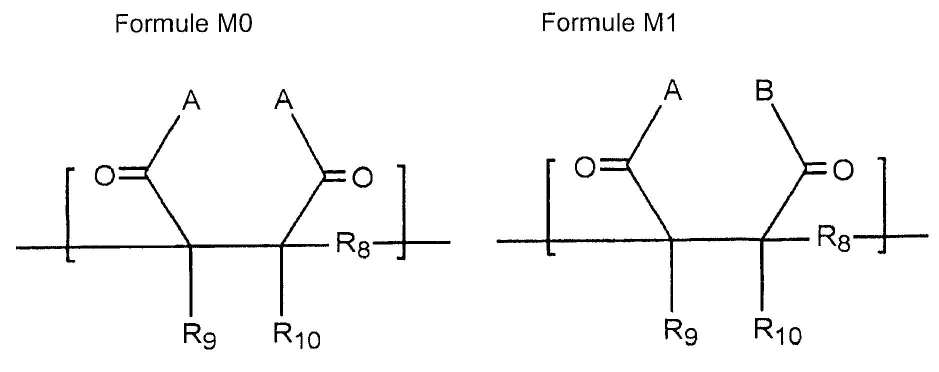 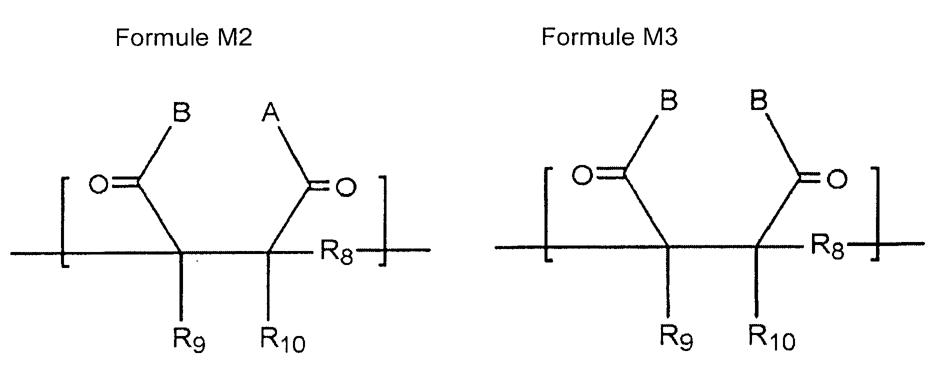 où A est -OH, B est 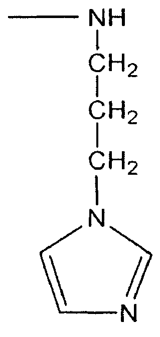 et
où R₈ est un groupement éthyle, et R₉ et R₁₀ sont des atomes d'hydrogène.

2. La méthode de la revendication 1, où la concentration du sel cosmotropique est comprise entre 0,3 et 3 M, et la concentration du sel chaotropique comprise entre 3 et 6 M.

3. La méthode de la revendication 1 ou 2, où le sel cosmotropique comprend un anion sélectionné dans un groupe constitué d'acétate, phosphate, sulfate et citrate, et un contre-cation de l'anion.

4. La méthode d'une des revendications 1 à 3, où le sel cosmotropique comporte un anion sélectionné dans un groupe constitué de Br⁻, NO₃⁻, I⁻, SCN- et Cl0₄⁻, et un contre-cation de l'anion.

5. La méthode d'une des revendications 1 à 4, où le rapport de concentrations molaires du sel cosmotropique au sel chaotropique est compris entre 1:14 et 1:2.

6. La méthode d'une des revendications 1 à 5, comprenant de plus la séparation de l'ADN plasmidique du mélange restant après séparation du support solide ou du surnageant par
mise en contact du surnageant avec un oxyde métallique,
séparation de l'oxyde métallique du surnageant et
élution de l'ADN plasmidique de l'oxyde métallique séparé.

7. La méthode de la revendication 6, où la séparation de l'oxyde métallique du mélange comprend une centrifugation du mélange ; et l'élimination du surnageant.

8. La méthode d'une des revendications 1 à 7, où le matériau du support solide comprend de plus un oxyde métallique, cet oxyde métallique étant de préférence un matériau à base de silice.

9. L'utilisation d'un matériel d'analyse pour la séparation de l'ADN génomique de l'ADN plasmidique dans un échantillon comprenant
un sel cosmotropique,
un sel chaotropique et
un matériau se liant à l'ADN à un pH de 3 à 5, où le matériau est bifonctionnel, ce matériau bifonctionnel étant chargé positivement et lié aux acides nucléiques, à un premier pH allant de 2 à 5, et chargé négativement et relâche les acides nucléiques liés, à un deuxième pH allant de 7 à 12, **caractérisé en ce que** le matériau bifonctionnel est un polymère incluant au moins un monomètre sélectionné dans un groupe constitué des monomères représentés par la formule M0, M1, M2 et M3 ci-dessous, où le polymère inclut au moins un monomère comprenant un fragment A et au moins un monomère comprenant un fragment B : 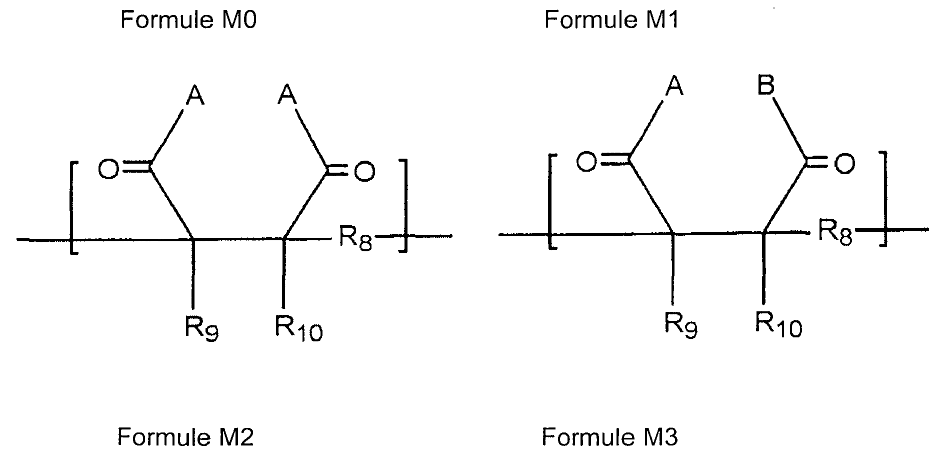 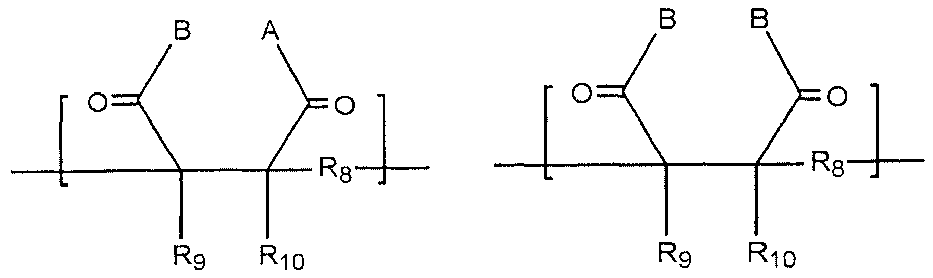 où A est -OH, B est 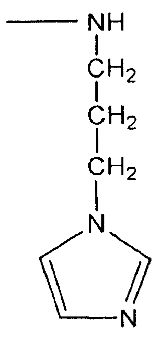 et
où R₈ est un groupement éthyle et R₉ et R₁₀ sont des atomes d'hydrogène.

10. L'utilisation de la revendication 9, où la concentration du sel cosmotropique est comprise entre 0,3 et 3 M, et la concentration du sel chaotropique comprise entre 3 et 6 M.

11. L'utilisation de la revendication 9 ou 10, où un matériel d'analyse comporte de plus un oxyde métallique, cet oxyde métallique étant de préférence un matériau à base de silice.
